# EUROPEAN PATENT APPLICATION

(11) **EP 4 817 585 A1**
(43) Date of publication of application: **07.10.2026**
(21) Application number: 26169724.7
(22) Date of filing: 31.03.2026
(51) Int. Cl.: A61H 31/00

(54) **MODIFYING CHEST COMPRESSION PARAMETERS TO REDUCE REFIBRILLATION RISK**

(30) Priority: 31.03.2025 US 202563781144 P; 26.03.2026 US 202619630039
(71) Applicant: STRYKER CORPORATION, Portage, MI 49002-9711 (US)
(72) Inventor: HARVEY, Adam, Portage, 49002-9711 (US); CHAPMAN, Fred W., Portage, 49002-9711 (US)
(74) Representative: Kilburn & Strode LLP

(57) **Abstract**

An example method includes administering first compressions to a chest of a subject at a first rate; identifying an electrocardiogram (ECG) of the subject; determining, by analyzing the ECG, that the subject has refibrillated; and in response to determining that the subject has refibrillated, administering second compressions to the chest of the subject at a second rate. The second rate is different than the first rate.

## Description

### CROSS-REFERENCE TO RELATED APPLICATIONS

This application claims priority to U.S. Provisional App. No. 63/781,144, which was filed on March 31, 2025, and to U.S. Nonprovisional App. No. 19/630,039, which was filed on March 26, 2026.

### BACKGROUND

Ventricular fibrillation (VF) is a serious cardiac arrhythmia that is treatable by administration of an electrical shock, a process referred to as "defibrillation." A defibrillator, for instance, can transition a subject's heart from a VF heart rhythm to a non-VF heart rhythm after administering an electrical shock to the subject's heart. However, in many cases, the subject's heart refibrillates, such that the VF recurs. Although the defibrillator can administer multiple electrical shocks to the subject in response to each instance of VF that occurs during a rescue event, the subject's likelihood of survival may be reduced as the number of electrical shocks administered to the subject increases.

### BRIEF DESCRIPTION OF THE DRAWINGS

FIG. 1 illustrates an example environment for modifying chest compression parameters in response to a condition of a subject.
FIG. 2 illustrates an example compressor configured to administer chest compressions at different contact areas.
FIG. 3 illustrates an example timing diagram for selectively modifying chest compression parameters based on a susceptibility to refibrillation.
FIG. 4 illustrates an example process for adjusting chest compressions to reduce a risk of refibrillation.
FIG. 5 illustrates an example process for adjusting chest compression parameters in response to the administration of multiple electrical shocks.
FIG. 6 illustrates an example of an external defibrillator configured to perform various functions described herein.
FIG. 7 illustrates a chest compression device configured to perform various functions described herein.

### DETAILED DESCRIPTION

Various implementations described herein relate to reducing the likelihood of refibrillation in a subject by selectively adjusting parameters of chest compressions during a rescue event. In some implementations, a device (e.g., a monitor-defibrillator or chest compression device) determines whether a subject receiving chest compressions has a sensitive condition that makes the subject particularly susceptible to refibrillation. Some evidence of the sensitive condition includes whether the subject has previously refibrillated, a number of electric shocks applied to the subject, the amount of time that the subject has remained in ventricular fibrillation (VF) or other cardiac arrhythmias, whether the subject has a relatively low-amplitude electrocardiogram (ECG), and other characteristics described herein. If the subject has the sensitive condition, the device may recommend or instruct a parameter adjustment to subsequent chest compressions applied to the subject. In particular examples, the chest compressions are adjusted to reduce the amount of mechanical energy transferred to the heart of the subject, which may reduce the likelihood that the chest compressions will refibrillate the heart of the subject. For instance, the subsequent chest compressions may have a lower frequency, a lower duty cycle, a greater contact area with the chest of the subject, a shallower depth, a lower speed, utilize active decompression, or make contact with the subject at a different location on the subject's chest. While, in some cases, the adjusted chest compressions may not be able to achieve maximal blood flow through the body of the subject as conventional chest compressions, they nonetheless may be preferable if the subject would otherwise refibrillate in response to the more conventional chest compressions.

Various implementations of the present disclosure are directed to specific improvements to the technical field of medical technology as applied to emergency care. Previously, rescuers and chest compression devices would apply chest compressions to a subject exhibiting VF at parameters to maximize blood flow through the body of a subject while the heart of the subject was unable to spontaneously pump blood throughout the body of the subject. However, such chest compressions could cause the subject to refibrillate after receiving an electric shock, which could reduce the likelihood that the subject would survive the rescue event. In various implementations of the present disclosure, the chest compression parameters can be adjusted to reduce the likelihood of refibrillation, which may increase the likelihood of survival after the rescue event.

Implementations of the present disclosure will now be described with reference to the accompanying figures.

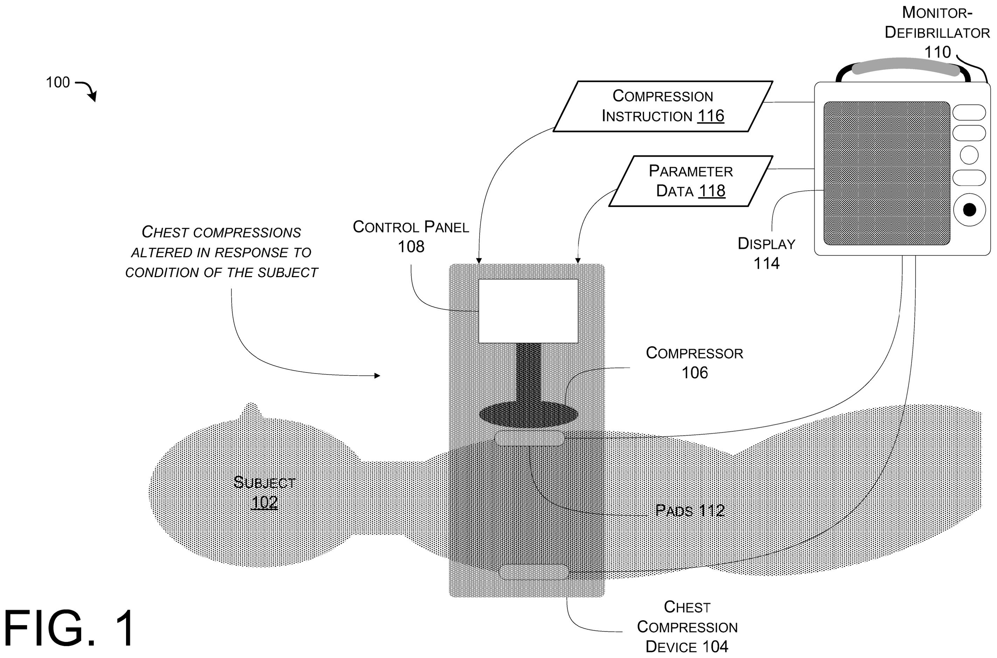

FIG. 1 illustrates an example environment 100 for modifying chest compression parameters in response to a condition of a subject 102. In various cases, the environment 100 includes a rescue scene in which the subject 102 is experiencing a medical emergency. In various cases, the environment 100 is a non-clinical environment, such as a setting outside of a hospital, medical clinic, hospice, or other clinical environment. For example, the environment 100 may include a setting in which the subject 102 originally experienced the medical emergency. In some examples, the subject 102 may have unexpectedly lost consciousness in the environment 100.

In various cases, the subject 102 is experiencing a medical condition that can be treated, at least in part, by administering chest compressions to the subject 102. In some implementations, the heart of the subject 102 is unable to pump blood sufficiently through the body of the subject 102. For instance, the subject 102 may be experiencing a cardiac arrhythmia, such as VF, pulseless ventricular tachycardia (VT), or some other condition in which the subject 102 lacks a pulse. While the heart of the subject is not spontaneously circulating blood through the body of the subject 102, various tissues within the body of the subject 102 may receive insufficient oxygen. In various cases, the subject 102 may be in danger of experiencing a hypoxic injury (e.g., to the brain or other vital organs) while the blood of the subject 102 is not spontaneously circulating through the body of the subject 102. In various cases, the application of chest compressions may at least temporarily push blood through the body of the subject 102, thereby preventing the subject 102 from experiencing a hypoxic injury when the heart of the subject 102 is unable to spontaneously pump blood through the body of the subject 102.

In some cases, chest compressions are applied to the subject 102 manually. For instance, a rescuer (not illustrated) may periodically compress the chest of the subject 102 with the rescuer's hands. However, manual chest compressions have some drawbacks. For example, if the rescuer experiences fatigue during manual chest compressions, the quality of the chest compressions may decrease over time.

In various implementations, a chest compression device 104 (also referred to as a "mechanical chest compression device") is utilized to administer chest compressions to the subject 102. In various implementations, the chest compression device 104 is a mechanical device configured to periodically compress the chest of the subject 102. According to some examples, the chest compression device 104 is a portable device powered by one or more batteries (e.g., one or more rechargeable batteries). In various cases, the chest compression device 104 includes a compressor 106 configured to be disposed on the chest of the subject 102. In some cases, the compressor 106 includes a plunger, a piston, or a belt configured to be applied to the chest of the subject 102. In various cases, the chest compression device 104 includes a piston or other mechanical actuator configured to cause the compressor 106 to squeeze the chest of the subject 102. In some cases, the compressor 106 is configured to adhere to the skin of the subject 102, and is further configured to administer active decompression by lifting the skin of the subject 102 between chest compressions. In some aspects, the compressor 106 adheres to the skin of the subject by a suction cup, adhesive, or a combination thereof.

The chest compression device 104 further includes a control panel 108 that is configured to detect input signals and to output signals relevant to the operation of the chest compression device 104. In some aspects, the control panel 108 includes at least one of a dial, a button, a switch, a keypad, a touch sensor, or a microphone configured to detect an input signal from the rescuer. In various cases, the chest compression device 104 is configured to perform one or more actions in response to the input signal. For instance, in response to the input signal, the chest compression device 104 may power on, move or tighten the compressor 106, pause chest compressions, or set and/or modify a parameter of the chest compressions applied by the chest compression device 104 to the subject 102. The rescuer, for example, controls the operation of the chest compression device 104 using the control panel 108.

The terms " parameter of the chest compressions," "chest compression parameter," "treatment parameter," and their equivalents, may refer to a metric characterizing applied chest compressions. Examples of chest compression parameters include a frequency of the chest compressions, a position of the chest compressions on the chest, a depth of the chest compressions, a duty cycle of the chest compressions, a speed of the chest compressions, a force of the chest compressions, a timing of the chest compressions, or any combination thereof. The frequency of the chest compressions may refer to a frequency at which the compressor 106 applies pressure to the chest of the subject 102. The position of the chest compressions may refer to a lateral location of the compressor 106 on the surface of the chest of the subject 102 while the chest compressions are applied. The depth of the chest compressions may refer to a distance between the chest of the subject 102 in an uncompressed state and the chest of the subject 102 in a compressed state. The duty cycle of the chest compressions may refer to a fraction or percentage of time that the compressor 106 is actively applying pressure to the chest of the subject 102 with respect to a total amount of time that the chest compressions are being applied. The speed of the chest compressions may refer to the speed at which a compressor applying the chest compression lowers onto the chest of the subject and/or a speed at which the compressor raises after applying each compression.

In various implementations, the control panel 108 includes at least one output device, such as a display (e.g., a screen), a light source, a speaker, or the like, configured to report information relevant to the operation of the chest compression device 104 to the rescuer. In some cases, the control panel 108 indicates a current power status (e.g., battery charge level) of the chest compression device 104. The control panel 108, in some aspects, indicates errors in operation of the chest compression device 104. In some cases, the control panel 108 indicates when one or more components (e.g., the compressor 106) of the chest compression device 104 are ready to be replaced. In some examples, the control panel 108 indicates whether the chest compression device 104 is powered on or off. In various implementations, the control panel 108 indicates whether chest compressions are currently being applied to the subject 102 or whether the chest compressions are paused. According to some implementations, the control panel 108 indicates one or more chest compression parameters of the chest compressions being applied to the subject 102 by the chest compression device 104.

Other medical devices may be present to facilitate management and treatment of the condition of the subject 102. As illustrated in FIG. 1, a monitor-defibrillator 110 is further included in the environment 100. In various cases, the monitor-defibrillator 110 is configured to detect one or more physiological parameters of the subject. Examples of physiological parameters include an ECG, an electroencephalogram (EEG), an electrical impedance (e.g., a transthoracic impedance), a heart rate, a pulse rate, a blood flow parameter (e.g., a velocity or volumetric flow rate of blood through one or more blood vessels), a blood vessel shape (e.g., a cross-sectional area of one or more blood vessels), a blood oxygenation (e.g., a pulse oxygenation (SpO₂), a regional oxygenation, a cerebral oxygenation, a photoplethysmography, etc.), an airway parameter (e.g., a flow of air in an airway, a pressure of air in the airway, a partial pressure of one or more gasses (e.g., CO₂, O₂, etc.) in the airway, a respiration rate, a ventilation rate, etc.), a capnograph, an end-tidal CO₂ (EtCO₂), a blood pressure (e.g., a systolic blood pressure, a diastolic blood pressure, an instantaneous blood pressure, etc.), a temperature (e.g., a core temperature), or any combination thereof. In some cases, the monitor-defibrillator 110 is configured to detect other parameters, such as an acceleration, orientation, or movement of the subject 102. For instance, the monitor-defibrillator 110 includes, or is communicatively coupled with, one or more sensors such as one or more electrodes, a heart rate monitor, a pulse sensor, an interferometric sensor, an ultrasound transducer, a medical imaging device, an oxygenation sensor (e.g., including a light source and a light detector), a flow sensor, a pressure sensor, a gas sensor, a blood pressure sensor, a thermometer, an accelerometer, a gyroscope, or any combination thereof.

In particular implementations, the monitor-defibrillator 110 is communicatively and/or electrically coupled with pads 112 configured to be adhered to the skin of the subject 102. In various cases, the pads 112 include electrodes that are configured to detect an electrical signal from the heart of the subject 102 over time and/or to output an electrotherapy from the monitor-defibrillator 110 to the heart of the subject 102. According to various cases, the electrical signal is indicative of the ECG of the subject 102, such that the monitor-defibrillator 110 is configured to detect the ECG of the subject 102 via the pads 112. Optionally, the monitor-defibrillator 110 is further configured to detect an impedance (e.g., transthoracic impedance) of the subject 102 using the electrodes of the pads 112. In some implementations, the monitor-defibrillator 110 is configured to output an indication of the ECG and/or other physiological parameters of the subject 102 using a display 114.

In some aspects, the monitor-defibrillator 110 is configured to assess the condition of the subject 102 by performing an analysis on the ECG and/or the other physiological parameters. For instance, the monitor-defibrillator 110 may be configured to determine whether the subject 102 has an arrhythmia (e.g., VF, bradycardia, or the like) by analyzing the ECG, to determine whether the subject 102 has spontaneous circulation by analyzing parameters indicative of blood flow, or the like. In some examples, the monitor-defibrillator 110 generates and/or outputs an indication of the determined condition of the subject 102. In some cases, the monitor-defibrillator 110 generates and/or outputs a recommendation to administer a treatment to the subject 102. For instance, the monitor-defibrillator 110 may output a recommendation to administer the electrotherapy (e.g., one or more electrical shocks, pacing pulses, synchronized cardioversion, or the like) via the display 114. In various cases, the monitor-defibrillator 110 further outputs the electrotherapy to the subject 102, such as in response to receiving an input signal from the rescuer.

In various examples, both the chest compression device 104 and the monitor-defibrillator 110 enhance the condition of the subject 102. The chest compression device 104 may temporarily induce blood circulation in the body of the subject 102 while the subject is experiencing VF. Further, the monitor-defibrillator 110 is configured to treat the underlying VF arrhythmia using an electrotherapy and can thereby restart adequate blood circulation in the body of the subject 102.

However, in some cases, the application of chest compressions to the subject 102 can interfere in the treatment of the cardiac arrhythmia. The chest compressions applied by the chest compression device 104 mechanically squeeze the heart of the subject 102. In cases in which the heart has recently transitioned from a VF state to a non-VF state, such as in response to the administration of an electrical shock by the monitor-defibrillator 110, the heart may be temporarily unable to adequately circulate blood through the body of the subject 102 (or the rescuer may be unable to identify whether the heart is able to circulate blood through the body), such that continued chest compressions are warranted. However, the heart may also be susceptible to returning to a VF state based on the mechanical squeezing of the heart of the subject 102. That is, the mechanical energy applied to the heart by the chest compressions may cause the heart of the subject 102 to refibrillate. Although subsequent application of an additional electrical shock may cause the heart to return to the non-VF state, the survival of the subject 102 may be enhanced by limiting the amount of time that the subject 102 spends in the VF state and by limiting the number of electrical shocks administered to the subject 102.

In various implementations of the present disclosure, future refibrillation of the subject 102 may be prevented by selectively adjusting chest compression parameters in response to the condition of the subject 102. According to some examples, the chest compression device 104 administers first chest compressions characterized by one or more first chest compression parameters. In response to the condition of the subject 102, the chest compression device 104 may administer second chest compressions characterized by one or more second chest compression parameters, wherein the second chest compression parameter(s) are different than the first chest compression parameter(s).

In some examples, the monitor-defibrillator 110 determines whether the subject 102 has a condition that warrants adjustment of the chest compressions. The condition, also referred to herein as a "sensitive condition," is one that makes the subject 102 susceptible to chest compression-induced refibrillation. In some cases, the monitor-defibrillator 110 determines whether the subject 102 has the sensitive condition by determining whether the subject 102 has previously refibrillated. Previous episodes of refibrillation, in various cases, may be indicative that the heart of the subject 102 is sensitive to further episodes of refibrillation. In some cases, relatively weak heart rhythms (e.g., fine or low-amplitude VF) and/or relatively long periods of time in which the subject 102 is experiencing VF may also factor into a determination that the subject 102 has a sensitive condition.

In various implementations, the monitor-defibrillator 110 determines whether the subject 102 has the sensitive condition by analyzing one or more parameters (e.g., physiological parameter(s)) of the subject 102. In some cases, the monitor-defibrillator 110 determines whether the subject 102 has previously refibrillated by analyzing the ECG of the subject 102. In various implementations, the monitor-defibrillator 110 is configured to remove a chest compression artifact from the ECG before analysis. For instance, the monitor-defibrillator 110 may apply, to data indicative of the ECG, a comb filter rejecting the frequency of the chest compressions and one or more harmonics of the frequency.

In various cases, if the ECG of the subject 102 includes multiple segments indicative of VF, which may be separated by segments indicative of a non-VF heart rhythm, then the monitor-defibrillator 110 may infer that the subject 102 has previously refibrillated. For instance, the monitor-defibrillator 110 may determine that the ECG includes a transition into VF from a non-VF heart rhythm.

Other aspects of the ECG may be further indicative of whether the subject 102 has a sensitive condition. For example, the monitor-defibrillator 110 may determine that the subject 102 is susceptible to refibrillation by determining that an amplitude spectrum area (AMSA) and/or amplitude of the ECG is below a threshold. According to some cases, the monitor-defibrillator 110 determines that the subject 102 is susceptible to refibrillation by determining that the subject 102 has remained in VF for greater than a threshold amount of time. For instance, the monitor-defibrillator 110 may determine the amount of time that the subject 102 has remained in VF by analyzing the ECG.

In various cases, the monitor-defibrillator 110 infers that the subject 102 has previously refibrillated by determining that the subject 102 has received multiple electrical shocks over time. For instance, if the monitor-defibrillator 110 administers multiple electrical shocks to the subject 102, or detects multiple artifacts in the ECG indicating multiple electrical shocks administered to the subject 102, then the monitor-defibrillator 110 may infer that the subject 102 is susceptible to refibrillation.

In some aspects, the monitor-defibrillator 110 infers that the subject 102 has a sensitive condition by determining that the subject 102 has previously refibrillated when the subject 102 was receiving chest compressions from the chest compression device 104. In some cases, the monitor-defibrillator 110 detects the application of chest compressions by detecting, in at least one of the parameter(s), an artifact associated with the chest compressions. For example, the chest compressions can be detected by identifying periodic troughs in an impedance waveform of the subject 102. According to some cases, the monitor-defibrillator 110 infers that chest compressions are being applied when the monitor-defibrillator 110 has activated chest compression artifact filtering (e.g., of the ECG). In various implementations, the monitor-defibrillator 110 determines that the subject has a sensitive condition by determining that the subject has previously refibrillated within a threshold time period after the chest compressions (e.g., an initial chest compression after a pause, or the first chest compression applied to the subject 102) are applied to the subject 102. The threshold time period, for instance, may be 5 seconds, 10 seconds, 30 seconds, one minute, or the like. In various cases, the monitor-defibrillator 110 repeatedly (e.g., periodically) assesses whether the subject 102 has the sensitive condition.

In some implementations, the monitor-defibrillator 110 is configured to calculate a heart viability index based on one or more parameters of the subject 102. Based on the heart viability index, the monitor-defibrillator 110 may infer whether the subject 102 has a sensitive condition. For instance, the heart viability index may be a function of an amplitude (e.g., average amplitude during a time interval) of the ECG during VF, the AMSA during VF (e.g., during a time interval), the total amount of time that the subject 102 has remained in VF over the course of the rescue event, a blood pressure of the subject 102, an EtCO₂ of the subject 102, a blood oxygenation (e.g., SpO₂) of the subject 102, a number of shocks previously applied to the subject 102, or any combination thereof. In some implementations, the heart viability index is adjusted, in real-time, when the monitor-defibrillator 110 detects fibrillation or some other change in a state of the subject 102. In various cases, the heart viability index is indicative of the likelihood that a subsequent electrical shock will successfully defibrillate the subject 102 (e.g., permanently and/or temporarily) and result in the heart having a perfusing rhythm. For instance, the heart viability index may be proportional to the amplitude of the ECG, the AMSA, the blood pressure, the EtCO₂, the blood oxygenation, or any combination thereof. In some aspects, the heart viability index is inversely proportional to the number of shocks applied to the subject and/or the amount of time that the subject has remained in VF over the course of the rescue event. If the heart viability index, or a derivative of the heart viability index with respect to time, is below a threshold, then the monitor-defibrillator 110 may determine that the subject has the sensitive condition.

Other factors may weigh in favor of determining that the subject 102 has a sensitive condition susceptible to refibrillation. If the subject 102 has received a shock that has not only treated the VF, but has also induced spontaneous circulation in the subject 102, then the chest compressions may no longer be warranted. However, continued chest compressions can cause the subject 102 to reenter VF. In various cases, the monitor-defibrillator 110 determines whether the subject 102 has a perfusing rhythm in order to determine whether to pause or alter the chest compressions. Various techniques can be utilized to identify spontaneous circulation of the subject 102, such as by determining that a blood flow parameter, a blood pressure, blood oxygenation, or EtCO₂ of the subject has risen above a threshold. In some aspects, the monitor-defibrillator 110 infers that the shock successfully caused the heart to enter a perfusing rhythm. For instance, if the heart viability (e.g., AMSA) of the subject 102 is above a threshold prior to the shock, then the monitor-defibrillator 110 may infer that the shock has a high likelihood of inducing a perfusing rhythm. In this case, the monitor-defibrillator 110 may infer that the subject 102 has a sensitive condition, in that the likelihood of benefit of continued chest compressions after the shock at the same parameters may be outweighed by the likelihood of harm of the chest compressions.

In response to detecting that the subject 102 has the sensitive condition (or that the shock likely produced a perfusing rhythm), the monitor-defibrillator 110 may transmit a compression instruction 116 to the chest compression device 104. For example, the monitor-defibrillator 110 may transmit the compression instruction 116 as one or more communication signals over a wired and/or wireless communication interface. In various cases, the compression instruction 116 may specify one or more adjusted compression parameters, an indication that the subject 102 has the sensitive condition, an instruction to adjust one or more compression parameters applied by the chest compression device 104, or a combination thereof. Upon receiving the compression instruction 116, the chest compression device 104 may change one or more parameters of the chest compressions applied to the subject 102 to prevent the subject 102 from refibrillating.

Various types of chest compression parameter changes may reduce the likelihood that the subject 102 will refibrillate. For example, the likelihood of refibrillation due to the application of chest compressions may be reduced by lowering the rate of the chest compressions (e.g., reducing the frequency of the chest compressions from 100 compressions per minute to 80 compressions per minute), reducing a depth of the chest compressions during the compression phase, increasing a height of the chest compressions during a decompression phase, increasing a contact area of the compressor 106 on the chest of the subject 102, changing a contact location of the compressor 106 on the subject 102, reducing a duty cycle of the chest compressions (e.g., reducing the duty cycle from 0.5 to 0.3), changing a compression and/or decompression waveform (e.g., reducing a speed or acceleration of the compressor 106 when administering chest compressions, changing a waveform representing the position of the compressor 106 from a trapezoidal waveform to a sinusoidal waveform, etc.), or any combination thereof. In various cases, the second chest compressions applied to the subject 102 apply less energy to the heart of the subject 102 than the first chest compressions applied to the subject 102. In some examples, the second chest compressions with the adjusted chest compression parameter(s) are outside of conventional chest compression recommendations. For instance, it may be recommended that chest compressions are applied at a frequency of 100Hz, but in implementations of the present disclosure, a chest compression frequency can be lowered (e.g., to 50, 60, 70, 80, or 90 Hz). In some implementations, the monitor-defibrillator 110 causes the chest compression device 104 to initiate additional pauses in the chest compressions (e.g., to check for a pulse of the subject 102) in response to detecting the sensitive condition.

According to some examples, the depth or height of the chest compressions may be adjusted to reduce the likelihood of refibrillation. In various examples, a chest compression includes two phases: a compression phase during which a compressor (e.g., a piston or rescuer hands) is pressed into the chest, and a decompression phase in which the compressor is lifted from the chest. In some examples, the likelihood of refribrillation can be reduced by reducing the depth of the compressor during the compression phase. In some cases, the likelihood of refibrillation can be reduced by increasing the height of the chest during the decompression phase. In some examples, the compressor is adhered to the chest (e.g., via a suction cup), such that the compressor is capable of lifting the chest during the decompression phase. Lifting the chest during the decompression phase can be referred to as "active decompression." In some cases, the chest compressions are adjusted such that they are applied shallower and with a greater height (e.g., using active decompression), but such that the chest compressions retain the same vertical displacement as previously applied chest compressions.

In particular implementations, a timing of the chest compressions applied to the subject 102 is adjusted based on the detection of the sensitive condition. For example, in response to detecting the sensitive condition, the monitor-defibrillator 110 may cause the chest compression device 104 to refrain from administering the chest compressions at one or more vulnerable time periods of the heart of the subject 102. In particular cases, the subject 102 has an intrinsic cardiac cycle, as indicated by the ECG of the subject 102. In various implementations, chest compressions are more likely to induce fibrillation when they are applied during a vulnerable period of the cardiac cycle. In some examples, the vulnerable period includes a T-wave of the subject 102. In various implementations, the monitor-defibrillator 110 prevents the chest compression device 104 from administering chest compressions during a vulnerable period of the subject 102.

In some implementations, a location of the chest compressions applied to the subject 102 is adjusted based on the detection of the sensitive condition. In some aspects, the location of the chest compressions is adjusted from a first position on the chest to a second position on the chest. In some cases, the compressions are adjusted from a position on the chest to a different position on the body of the subject 102. For instance, the location of the compressions can be shifted from a position on the chest of the subject 102 to a position on the abdomen of the subject 102. After adjusting the location of the compressions, the compressions may be associated with a reduced likelihood of refibrillation.

According to some aspects, the chest compression device 104 determines whether the subject 102 has a sensitive condition that warrants adjustment of the chest compressions. For example, the monitor-defibrillator 110 may transmit, to the chest compression device 104, parameter data 118 encoding the parameter(s). In various cases, the chest compression device 104 infers the condition of the subject 102 by analyzing the parameter data 118. Further, in some implementations, the chest compression device 104 includes, or is communicatively coupled with, one or more sensors configured to detect the parameter(s) of the subject 102. Accordingly, in some implementations, the chest compression device 104 is a standalone device configured to automatically adjust the chest compressions based on whether the subject 102 has a sensitive condition.

Although FIG. 1 has been described primarily with respect to chest compressions applied by the chest compression device 104, implementations are not so limited. In some examples, the monitor-defibrillator 110 outputs a recommendation and/or instruction to adjust one or more parameters of manual chest compressions in response to detecting the sensitive condition. For instance, the monitor-defibrillator 110 may instruct a rescuer to administer chest compressions at a lower rate and/or compression depth in response to detecting a sensitive condition.

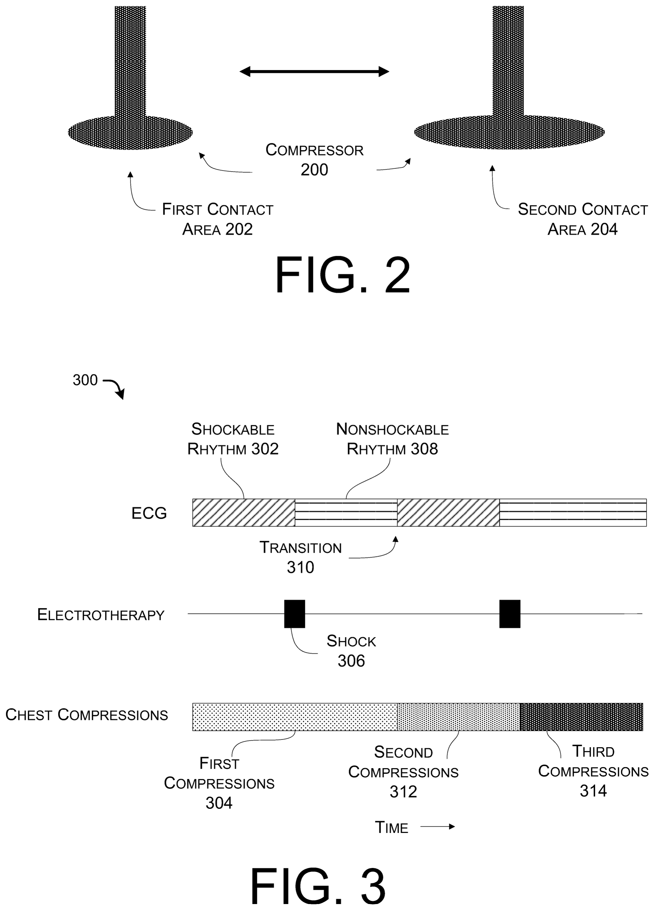

FIG. 2 illustrates an example compressor 200 configured to administer chest compressions at different contact areas. In a first state, the compressor 200 is configured to be disposed on a subject's chest via a first contact area 202. In a second state, the compressor 200 is configured to be disposed on the subject's chest via a second contact area 204. The second contact area 204 is larger than the first contact area 202. In various cases, the compressor 200 is part of, or communicatively coupled with, a chest compression device. According to some cases, a mechanism, circuit, actuator, or other active element within the compressor 200 selectively expands to the second state, or contracts to the first state, based on a control signal from the chest compression device.

Various types of elements can enable the compressor 200 to achieve the different contact areas. In some aspects, the compressor 200 includes a foldable portion. When the foldable portion is in a folded state, the compressor 200 has the first contact area 202. When the foldable portion is in a non-folded state, the compressor 200 has the second contact area 204. In various cases, the compressor 200 includes a hinge coupled with an actuator configured to selectively transition between the folded state and the non-folded state.

In some cases, the compressor 200 is inflatable. For example, the compressor 200 includes an elastic bladder that is configured to be filled with a fluid (e.g., air, water, or the like). When a first pressure of the fluid is disposed inside of the elastic bladder, the elastic bladder may have a first volume such that the compressor 200 has the first contact area 202. When a second pressure of the fluid is disposed inside of the elastic bladder, the elastic bladder may have a second volume such that the compressor 200 has the second contact area 204. In various cases, the second pressure is greater than the first pressure. The pressure of the fluid within the elastic bladder may be controlled by one or more valves, a pump, a fluid source (e.g., a gas tank), or any combination thereof. In some cases, a container of a first chemical may be disposed within the elastic bladder, and may be selectively punctured in order to introduce the first chemical to a second chemical in the elastic bladder. In some implementations, the first and second chemicals react in a chemical reaction (e.g., an exothermic reaction) that increases the pressure within the elastic bladder.

FIG. 3 illustrates an example timing diagram 300 for selectively modifying chest compression parameters based on a susceptibility to refibrillation. The timing diagram 300 indicates three channels corresponding to an ECG of a subject, an electrotherapy administered to the subject, and chest compressions applied to the subject over time. In the diagram 300, time increases from left to right, such that vertically aligned events occur at the same time.

In the time interval illustrated by the timing diagram 300, the ECG of the subject initially presents as a shockable rhythm 302. For instance, the shockable rhythm includes a cardiac arrhythmia that is treatable by an electric shock. Examples of the shockable rhythm include VF or pulseless VT. While the subject is exhibiting the shockable rhythm 302, the heart of the subject is unable to spontaneously and sufficiently circulate blood through the body of the subject. Accordingly, first compressions 304 are administered to the chest of the subject. The first compressions 304, in various cases, are characterized by one or more first chest compression parameters.

In various cases, a defibrillator administers a shock 306 to the heart of the subject. In various cases, the shock 306 is a defibrillating electrical shock. As a result of the shock 306, the ECG of the subject transitions from the shockable rhythm 302 to a nonshockable rhythm 308. In various cases, the nonshockable rhythm 308 is not treatable by an electric shock. The nonshockable rhythm 308, for instance, lacks indications of VF and/or VT. However, in various cases, the subject lacks sufficient spontaneous circulation (or it is unclear whether the subject has spontaneous circulation) of blood while exhibiting the nonshockable rhythm 308. Accordingly, the first compressions 304 continue while the ECG indicates the nonshockable rhythm 308.

According to some cases, the subject refibrillates such that the ECG of the subject returns to the shockable rhythm 302 after the nonshockable rhythm 308. In various cases, a transition 310 from the nonshockable rhythm 308 to the shockable rhythm 302 is indicated in the ECG. In some implementations, the first compressions 304 increase the likelihood of the transition 310 from the nonshockable rhythm 308 to the shockable rhythm 302. For instance, the transition 310 occurs, at least in part, in response to the first compressions 304.

In various cases, the chest compressions are adjusted in response to the transition 310. In some implementations, second compressions 312 are administered to the chest of the subject in response to detection of the transition 310. The second compressions 312 are characterized by at least one second chest compression parameter. In some examples, the second chest compression parameter(s) is different than the first chest compression parameter(s). For instance, the second compressions 312 have a lower frequency, a shallower depth, a greater contact area, a lower speed, a lower force, a different waveform (e.g., a force, depth, or position of the second compressions 312 has a sinusoidal waveform, whereas the first compressions 304 have a trapezoidal waveform), or a lower duty cycle than the first compressions 304. In some examples, the second compressions 312 utilize active decompression.

According to some cases, a second shock 306 is administered to the subject, thereby causing the ECG of the subject to transition from the shockable rhythm 302 to the nonshockable rhythm 308. In various cases, detection of the second shock 306 causes an inference that the subject has refibrillated. In various implementations, third compressions 314, characterized by at least one third chest compression parameter, are administered to the subject in response to the second instance of the shock 306. In some aspects, the third chest compression parameter(s) is different than the first chest compression parameter(s). For instance, the third compressions 314 have a lower frequency, a shallower depth, a greater contact area, a lower speed, a lower force, a different waveform, or a lower duty cycle than the first compressions 304. The third compressions 314, in some cases, utilize active decompression. In some cases, the third chest compression parameter(s) is different than the second chest compression parameter(s). For example, the third compressions 314 have a lower frequency, a shallower depth, a greater contact area, or a lower duty cycle than the second compressions 312. In some instances, the third compressions 314 utilize active decompression.

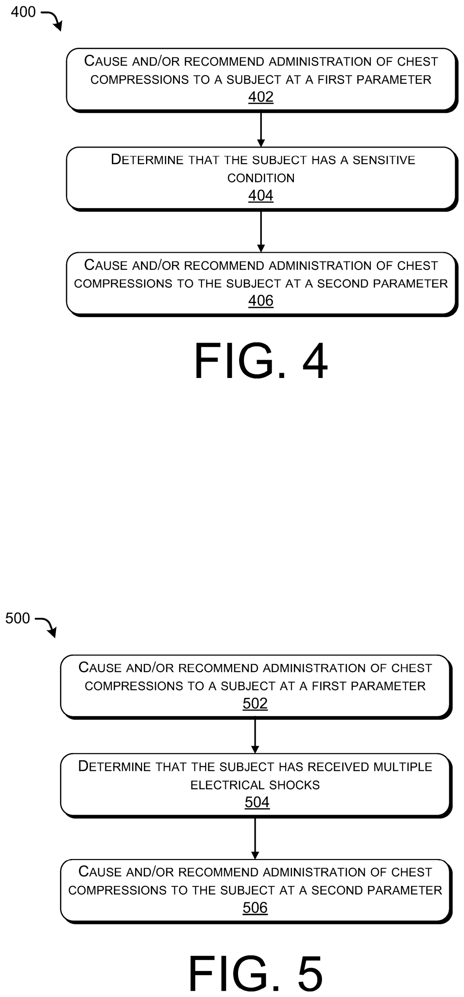

FIG. 4 illustrates an example process 400 for adjusting chest compressions to reduce a risk of refibrillation. The process 400 is performed by an entity, such as the chest compression device 104, the compressor 106, the monitor-defibrillator 110, the compressor 200, a medical device, at least one computing device, at least one processor, or any combination thereof.

At 402, the entity causes and/or recommends administration of chest compressions to a subject at a first parameter. In some implementations, the entity administers the chest compressions to the subject at the first parameter. In some implementations, the first parameter is a first rate, a first compression depth, a first location on the body of the subject, a first duty cycle, a first contact area, a first speed, a first force, a first waveform, or a combination thereof.

At 404, the entity determines that the subject has a sensitive condition. For example, the entity may determine that the subject has previously refibrillated. In some aspects, the entity analyzes an ECG of the subject in order to determine whether the subject has the sensitive condition. For instance, the entity may determine that the ECG is indicative of a transition into VF from a non-VF heart rhythm. In some cases, the entity determines that an AMSA of the ECG is below a threshold and/or that an average amplitude of the ECG is below a threshold. In some cases, the entity determines that the transition into VF from the non-VF heart rhythm occurred within a threshold time period after at least one of the chest compressions at the first parameter were administered to the subject. In particular cases, the entity administers one or more electrical shocks in response to detecting the VF indicated by the ECG of the subject.

At 406, the entity causes and/or recommends administration of chest compressions to the subject at a second parameter. In some cases, the entity administers the chest compressions at the second parameter. The second parameter is different than the first parameter, for instance. For example, the second parameter is a second rate, a second compression depth, a second location on the body of the subject (e.g., from chest to abdomen, from one position on the chest to a different position on the chest, etc.), a second duty cycle, a second contact area, a second speed, a second force, a second waveform, or a combination thereof. In various cases, the second rate is lower than the first rate, the second compression depth is shorter than the first compression depth (the first compression depth is longer than the second compression depth), the second location is farther from a center of the chest than the first location, the second duty cycle is smaller than the first duty cycle (the first duty cycle is higher than the second duty cycle), the second contact area is larger than the first contact area, or any combination thereof.

FIG. 5 illustrates an example process 400 for adjusting chest compression parameters in response to the administration of multiple shocks. The process 500 is performed by an entity, such as the chest compression device 104, the compressor 106, the monitor-defibrillator 110, the compressor 200, a medical device, at least one computing device, at least one processor, or any combination thereof.

At 502, the entity causes and/or recommends administration of chest compressions to a subject at a first parameter. In some implementations, the entity administers the chest compressions to the subject at the first parameter. In some implementations, the first parameter is a first rate, a first compression depth, a first location on the body of the subject, a first duty cycle, a first contact area, a first speed, a first force, a first waveform, or a combination thereof.

At 504, the entity determines that the subject has received multiple electrical shocks. In some implementations, the entity itself administers the multiple electrical shocks to the subject. In some cases, the entity detects administration of the multiple electrical shocks by detecting an artifact associated with the electrical shocks in data indicating a parameter (e.g., physiological parameter) detected from the subject. In some examples, the entity receives, from an external device, one or more communication signals indicating the administration of the multiple electrical shocks.

At 506, the entity causes and/or recommends administration of chest compressions to the subject at a second parameter. In some cases, the entity administers the chest compressions at the second parameter. The second parameter is different than the first parameter, for instance. For example, the second parameter is a second rate, a second compression depth, a second location on the body of the subject (e.g., from chest to abdomen, from one position on the chest to a different position on the chest, etc.), a second duty cycle, a second contact area, a second speed, a second force, a second waveform, or a combination thereof. In various cases, the second rate is lower than the first rate, the second compression depth is shorter than the first compression depth, the second location is farther from a center of the chest than the first location, the second duty cycle is smaller than the first duty cycle, the second contact area is larger than the first contact area, or any combination thereof.

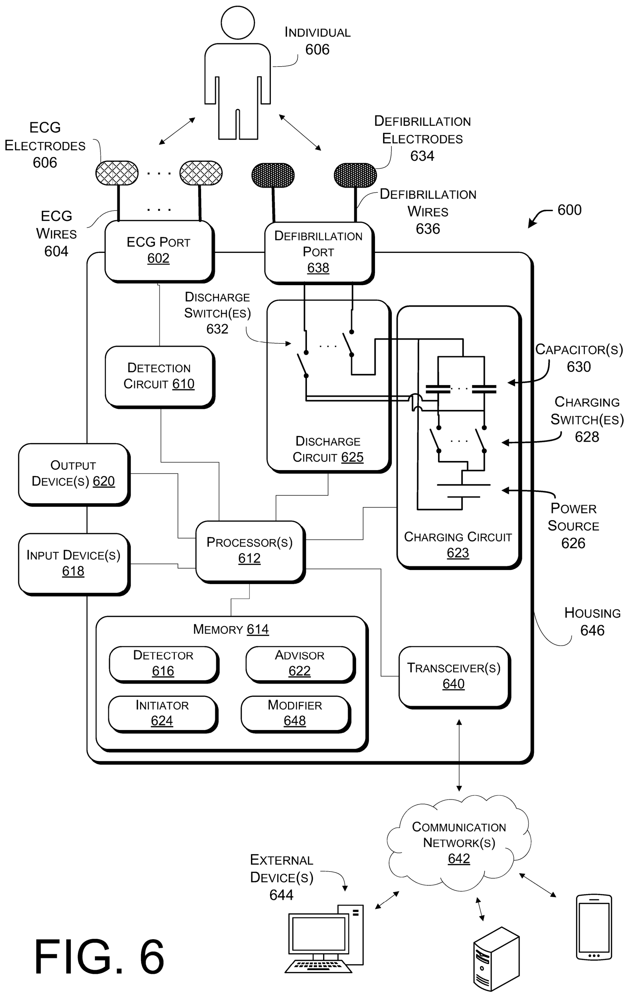

FIG. 6 illustrates an example of an external defibrillator 600 configured to perform various functions described herein. For example, the external defibrillator 600 is the monitor-defibrillator 110 described above with reference to FIG. 1.

The external defibrillator 600 includes an ECG port 602 connected to multiple ECG wires 604. In some cases, the ECG wires 604 are removeable from the ECG port 602. For instance, the ECG wires 604 are plugged into the ECG port 602 via connectors. The ECG wires 604 are connected to ECG electrodes 606, respectively. In various implementations, the ECG electrodes 606 are disposed on different locations on an individual 608 (also referred to as a "subject"). A detection circuit 610 is configured to detect relative voltages between the ECG electrodes 606. These voltages are indicative of the electrical activity of the heart of the individual 608.

In various implementations, the ECG electrodes 606 are in contact with the different locations on the skin of the individual 608. In some examples, a first one of the ECG electrodes 606 is placed on the skin between the heart and right arm of the individual 608, a second one of the ECG electrodes 606 is placed on the skin between the heart and left arm of the individual 608, and a third one of the ECG electrodes 606 is placed on the skin between the heart and a leg (either the left leg or the right leg) of the individual 608. In these examples, the detection circuit 610 is configured to measure the relative voltages between the first, second, and third ECG electrodes 606. Respective pairings of the ECG electrodes 606 are referred to as "leads," and the voltages between the pairs of ECG electrodes 606 are known as "lead voltages." In some examples, more than three ECG electrodes 606 are included, such that 5-lead or 12-lead ECG signals are detected by the detection circuit 610.

The detection circuit 610 includes at least one analog circuit, at least one digital circuit, or a combination thereof. The detection circuit 610 receives the analog electrical signals from the ECG electrodes 606, via the ECG port 602 and the ECG wires 604. In some cases, the detection circuit 610 includes one or more analog filters configured to filter noise and/or artifact from the electrical signals. The detection circuit 610 includes an analog-to-digital (ADC) in various examples. The detection circuit 610 generates a digital signal indicative of the analog electrical signals from the ECG electrodes 606. This digital signal can be referred to as an "ECG signal" or an "ECG."

In some cases, the detection circuit 610 further detects an electrical impedance between at least one pair of the ECG electrodes 606. For example, the detection circuit 610 includes, or otherwise controls, a power source that applies a known voltage (or current) across a pair of the ECG electrodes 606 and detects a resultant current (or voltage) between the pair of the ECG electrodes 606. The impedance is generated based on the applied signal (voltage or current) and the resultant signal (current or voltage). In various cases, variations in the impedance corresponds to respiration of the individual 608, chest compressions performed on the individual 608, and other physiological states of the individual 608. In various examples, the detection circuit 610 includes one or more analog filters configured to filter noise and/or artifact from the resultant signal. The detection circuit 610 generates a digital signal indicative of the impedance using an ADC. This digital signal can be referred to as an "impedance signal" or an "impedance."

The detection circuit 610 provides the ECG signal and/or the impedance signal to one or more processors 612 in the external defibrillator 600. In some implementations, the processor(s) 612 includes a central processing unit (CPU), a graphics processing unit (GPU), both CPU and GPU, or other processing unit or component known in the art.

The processor(s) 612 is operably connected to memory 614. In various implementations, the memory 614 is volatile (such as random access memory (RAM)), nonvolatile (such as read only memory (ROM), flash memory, etc.) or some combination of the two. The memory 614 stores instructions that, when executed by the processor(s) 612, causes the processor(s) 612 to perform various operations. In various examples, the memory 614 stores methods, threads, processes, applications, objects, modules, any other sort of executable instruction, or a combination thereof. In some cases, the memory 614 stores files, databases, or a combination thereof. In some examples, the memory 614 includes, but is not limited to, RAM, ROM, electrically erasable programmable read-only memory (EEPROM), flash memory, or any other memory technology. In some examples, the memory 614 includes one or more of CD-ROMs, digital versatile discs (DVDs), content-addressable memory (CAM), or other optical storage, magnetic cassettes, magnetic tape, magnetic disk storage or other magnetic storage devices, or any other medium which can be used to store the desired information and which can be accessed by the processor(s) 612 and/or the external defibrillator 600. In some cases, the memory 614 at least temporarily stores the ECG signal and/or the impedance signal.

In various examples, the memory 614 includes a detector 616, which causes the processor(s) 612 to determine, based on the ECG signal and/or the impedance signal, whether the individual 608 is exhibiting a particular heart rhythm. For instance, the processor(s) 612 determines whether the individual 608 is experiencing a shockable rhythm that is treatable by defibrillation. Examples of shockable rhythms include VF and pulseless VT. In some examples, the processor(s) 612 determines whether any of a variety of different rhythms (e.g., asystole, sinus rhythm, atrial fibrillation (AF), etc.) are present in the ECG signal.

The processor(s) 612 is operably connected to one or more input devices 618 and one or more output devices 620. Collectively, the input device(s) 618 and the output device(s) 620 function as an interface between a user (e.g., a rescuer) and the defibrillator 600. The input device(s) 618 is configured to receive an input from a user and includes at least one of a keypad, a cursor control, a touch-sensitive display, a voice input device (e.g., a microphone), a haptic feedback device (e.g., a gyroscope), or any combination thereof. The output device(s) 620 includes at least one of a display, a speaker, a haptic output device, a printer, or any combination thereof. In various examples, the processor(s) 612 causes a display among the input device(s) 618 to visually output a waveform of the ECG signal and/or the impedance signal. In some implementations, the input device(s) 618 includes one or more touch sensors, the output device(s) 620 includes a display screen, and the touch sensor(s) are integrated with the display screen. Thus, in some cases, the external defibrillator 600 includes a touchscreen configured to receive user input signal(s) and visually output physiological parameters, such as the ECG signal and/or the impedance signal.

In various implementations, the input device(s) 618 further include, or are otherwise connected to, one or more physiological sensors. The physiological sensor(s), for instance, are configured to detect one or more physiological parameters of the individual 608. Examples of the physiological sensor(s) include a blood pressure sensor (e.g., a blood pressure cuff, invasive blood pressure sensor, or the like), an airway sensor (e.g., a sensor configured to detect a partial pressure of CO₂ and/or O₂ in an airway of the individual 608), a blood oxygenation sensor (e.g., a pulse oximeter, regional oxygenation sensor, or the like), a thermometer, a pulse sensor, a blood flow sensor (e.g., an ultrasound transducer configured to detect blood flow using Doppler-based techniques), an airway pressure sensor, or any combination thereof. The input device(s) 618, in some cases, includes one or more sensors configured to detect other characteristics of the individual 608. For example, the input device(s) 618 includes an accelerometer, gyroscope, microphone, or any combination thereof. In various implementations, the processor(s) 612 is configured to assess a condition of the individual 608 by analyzing data derived from signals detected by the input device(s) 618.

In some examples, the memory 614 includes an advisor 622, which, when executed by the processor(s) 612, causes the processor(s) 612 to generate advice and/or control the output device(s) 620 to output the advice to a user (e.g., a rescuer). In some examples, the processor(s) 612 provides, or causes the output device(s) 620 to provide, an instruction to perform CPR on the individual 608. In some cases, the processor(s) 612 evaluates, based on the ECG signal, the impedance signal, or other physiological parameters, CPR being performed on the individual 608 and causes the output device(s) 620 to provide feedback about the CPR in the instruction. According to some examples, the processor(s) 612, upon identifying that a shockable rhythm is present in the ECG signal, causes the output device(s) 620 to output an instruction and/or recommendation to administer a defibrillation shock to the individual 608.

The memory 614 also includes an initiator 624 which, when executed by the processor(s) 612, causes the processor(s) 612 to control other elements of the external defibrillator 600 in order to administer a defibrillation shock to the individual 608. In some examples, the processor(s) 612 executing the initiator 624 selectively causes the administration of the defibrillation shock based on determining that the individual 608 is exhibiting the shockable rhythm and/or based on an input from a user (received, e.g., by the input device(s) 618. In some cases, the processor(s) 612 causes the defibrillation shock to be output at a particular time, which is determined by the processor(s) 612 based on the ECG signal and/or the impedance signal.

The processor(s) 612 is operably connected to a charging circuit 623 and a discharge circuit 625. In various implementations, the charging circuit 623 includes a power source 626, one or more charging switches 628, and one or more capacitors 630. The power source 626 includes, for instance, a battery. The processor(s) 612 initiates a defibrillation shock by causing the power source 626 to charge at least one capacitor among the capacitor(s) 630. For example, the processor(s) 612 activates at least one of the charging switch(es) 628 in the charging circuit 623 to complete a first circuit connecting the power source 626 and the capacitor to be charged. Then, the processor(s) 612 causes the discharge circuit 625 to discharge energy stored in the charged capacitor across a pair of defibrillation electrodes 634, which are in contact with the individual 608. For example, the processor(s) 612 deactivates the charging switch(es) 628 completing the first circuit between the capacitor(s) 630 and the power source 626, and activates one or more discharge switches 632 completing a second circuit connecting the charged capacitor 630 and at least a portion of the individual 608 disposed between defibrillation electrodes 634.

The energy is discharged from the defibrillation electrodes 634 in the form of a defibrillation shock. For example, the defibrillation electrodes 634 are connected to the skin of the individual 608 and located at positions on different sides of the heart of the individual 608, such that the defibrillation shock is applied across the heart of the individual 608. The defibrillation shock, in various examples, depolarizes a significant number of heart cells in a short amount of time. The defibrillation shock, for example, interrupts the propagation of the shockable rhythm (e.g., VF or VT) through the heart. In some examples, the defibrillation shock is 200 J or greater with a duration of about 0.015 seconds. In some cases, the defibrillation shock has a multiphasic (e.g., biphasic) waveform. The discharge switch(es) 632 are controlled by the processor(s) 612, for example. In various implementations, the defibrillation electrodes 634 are connected to defibrillation leads 636. The defibrillation wires 636 are connected to a defibrillation port 638, in implementations. According to various examples, the defibrillation wires 636 are removable from the defibrillation port 638. For example, the defibrillation wires 636 are plugged into the defibrillation port 638.

In various implementations, the processor(s) 612 is operably connected to one or more transceivers 640 that transmit and/or receive data over one or more communication networks 642. For example, the transceiver(s) 640 includes a network interface card (NIC), a network adapter, a local area network (LAN) adapter, or a physical, virtual, or logical address to connect to the various external devices and/or systems. In various examples, the transceiver(s) 640 includes any sort of wireless transceivers capable of engaging in wireless communication (e.g., radio frequency (RF) communication). For example, the communication network(s) 642 includes one or more wireless networks that include a 3^{rd} Generation Partnership Project (3GPP) network, such as a Long Term Evolution (LTE) radio access network (RAN) (e.g., over one or more LTE bands), a New Radio (NR) RAN (e.g., over one or more NR bands), or a combination thereof. In some cases, the transceiver(s) 640 includes other wireless modems, such as a modem for engaging in WI-FI^{®}, WIGIG^{®}, WIMAX^{®}, BLUETOOTH^{®}, or infrared communication over the communication network(s) 642.

The defibrillator 600 is configured to transmit and/or receive data (e.g., ECG data, impedance data, data indicative of one or more detected heart rhythms of the individual 608, data indicative of one or more defibrillation shocks administered to the individual 608, etc.) with one or more external devices 644 via the communication network(s) 642. The external devices 644 include, for instance, mobile devices (e.g., mobile phones, smart watches, etc.), Internet of Things (IoT) devices, medical devices, computers (e.g., laptop devices, servers, etc.), or any other type of computing device configured to communicate over the communication network(s) 642. In some examples, the external device(s) 644 is located remotely from the defibrillator 600, such as at a remote clinical environment (e.g., a hospital). According to various implementations, the processor(s) 612 causes the transceiver(s) 640 to transmit data to the external device(s) 644. In some cases, the transceiver(s) 640 receives data from the external device(s) 644 and the transceiver(s) 640 provide the received data to the processor(s) 612 for further analysis.

In various implementations, the external defibrillator 600 also includes a housing 646 that at least partially encloses other elements of the external defibrillator 600. For example, the housing 646 encloses the detection circuit 610, the processor(s) 612, the memory 614, the charging circuit 623, the transceiver(s) 640, or any combination thereof. In some cases, the input device(s) 618 and output device(s) 620 extend from an interior space at least partially surrounded by the housing 646 through a wall of the housing 646. In various examples, the housing 646 acts as a barrier to moisture, electrical interference, and/or dust, thereby protecting various components in the external defibrillator 600 from damage.

In some implementations, the external defibrillator 600 is an automated external defibrillator (AED) operated by an untrained user (e.g., a bystander, layperson, etc.) and can be operated in an automatic mode. In automatic mode, the processor(s) 612 automatically identifies a rhythm in the ECG signal, makes a decision whether to administer a defibrillation shock, charges the capacitor(s) 630, discharges the capacitor(s) 630, or any combination thereof. In some cases, the processor(s) 612 controls the output device(s) 620 to output (e.g., display) a simplified user interface to the untrained user. For example, the processor(s) 612 refrains from causing the output device(s) 620 to display a waveform of the ECG signal and/or the impedance signal to the untrained user, in order to simplify operation of the external defibrillator 600.

In some examples, the external defibrillator 600 is a monitor-defibrillator utilized by a trained user (e.g., a clinician, an emergency responder, etc.) and can be operated in a manual mode or the automatic mode. When the external defibrillator 600 operates in manual mode, the processor(s) 612 cause the output device(s) 620 to display a variety of information that may be relevant to the trained user, such as waveforms indicating the ECG data and/or impedance data, notifications about detected heart rhythms, and the like.

In some implementations, the memory 614 includes a modifier 648 that, when executed by the processor(s) 612, causes the processor(s) 612 to change a parameter of chest compressions applied to the individual 608. For instance, the modifier 648 includes instructions for identifying a sensitive condition of the individual 608 and/or generating an instruction to change a chest compression parameter. The instruction, for instance, is output to a rescuer via the output device(s) 620 and/or output to an external chest compression device via the transceiver(s) 640.

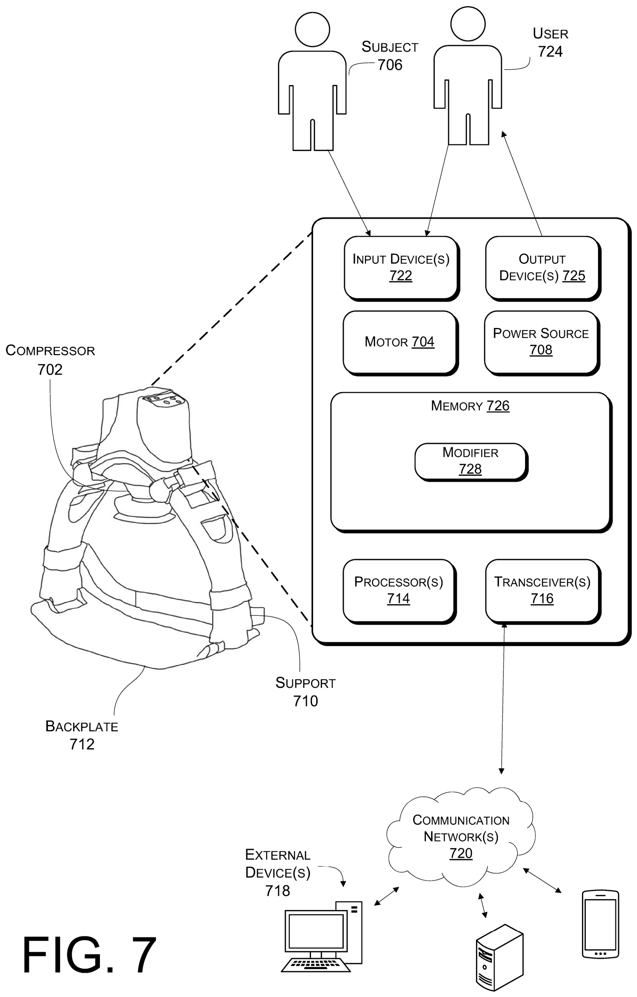

FIG. 7 illustrates a chest compression device 700 configured to perform various functions described herein. For example, the chest compression device 700 is the chest compression device 104 described with reference to FIG. 1.

In various implementations, the chest compression device 700 includes a compressor 702 that is operatively coupled to a motor 704. The compressor 702 physically administers a force to the chest of a subject 706 that compresses the chest of the subject 706. In some examples, the compressor 702 includes at least one piston that periodically moves between two positions (e.g., a compressed position and a release position) at a compression frequency. For example, when the piston is positioned on the chest of the subject 706, the piston compresses the chest when the piston is moved into the compressed position. A suction cup may be positioned on a tip of the piston, such that the suction cup contacts the chest of the subject 706 during operation. In various cases, the compressor 702 includes a band that periodically tightens to a first tension and loosens to a second tension at a compression frequency. For instance, when the band is disposed around the chest of the subject 706, the band compresses the chest when the band tightens.

The motor 704 is configured to convert electrical energy stored in a power source 708 into mechanical energy that moves and/or tightens the compressor 702, thereby causing the compressor 702 to administer the force to the chest of the subject 706. In various implementations, the power source 708 is portable. For instance, the power source 708 includes at least one rechargeable (e.g., lithium-ion) battery. In some cases, the power source 708 supplies electrical energy to one or more elements of the chest compression device 700 described herein.

In various cases, the chest compression device 700 includes a support 710 that is physically coupled to the compressor 702, such that the compressor 702 maintains a position relative to the subject 706 during operation. In some implementations, the support 710 is physically coupled to a backplate 712, cot, or other external structure with a fixed position relative to the subject 706. According to some cases, the support 710 is physically coupled to a portion of the subject 706, such as wrists of the subject 706.

The operation of the chest compression device 700 may be controlled by at least one processor 714. In various implementations, the motor 704 is communicatively coupled to the processor(s) 714. Specifically, the processor(s) 714 is configured to output a control signal to the motor 704 that causes the motor 704 to actuate the compressor 702. For instance, the motor 704 causes the compressor 702 to administer the compressions to the subject 706 based on the control signal. In some cases, the control signal indicates one or more treatment parameters of the compressions. Examples of treatment parameters include a frequency, timing, depth, force, position, velocity, and acceleration of the compressor 702 administering the compressions. According to various cases, the control signal causes the motor 704 to cease compressions.

In various implementations, the chest compression device 700 includes at least one transceiver 716 configured to communicate with at least one external device 718 over one or more communication networks 720. Any communication network described herein can be included in the communication network(s) 720 illustrated in FIG. 7. The external device(s) 718, for example, includes at least one of a monitor-defibrillator, an AED, an ECMO device, a ventilation device, a patient monitor, a mobile phone, a server, or a computing device. In some implementations, the transceiver(s) 716 is configured to communicate with the external device(s) 718 by transmitting and/or receiving signals wirelessly. For example, the transceiver(s) 716 includes a NIC, a network adapter, a LAN adapter, or a physical, virtual, or logical address to connect to the various external devices and/or systems. In various examples, the transceiver(s) 716 includes any sort of wireless transceivers capable of engaging in wireless communication (e.g., RF communication). For example, the communication network(s) 720 includes one or more wireless networks that include a 3GPP network, such as an LTE RAN (e.g., over one or more LTE bands), an NR RAN (e.g., over one or more NR bands), or a combination thereof. In some cases, the transceiver(s) 716 includes other wireless modems, such as a modem for engaging in WI-FI^{®}, WIGIG^{®}, WIMAX^{®}, BLUETOOTH^{®}, or infrared communication over the communication network(s) 720. The signals, in various cases, encode data in the form of data packets, datagrams, or the like. In some cases, the signals are transmitted as compressions are being administered by the chest compression device 700 (e.g., for real-time feedback by the external device(s) 718), after compressions are administered by the chest compression device 700 (e.g., for post-event review at the external device 718), or a combination thereof.

In various cases, the processor(s) 714 generates the control signal based on data encoded in the signals received from the external device(s) 718. For instance, the signals include an instruction to initiate the compressions, and the processor(s) 714 instructs the motor 704 to begin actuating the compressor 702 in accordance with the signals.

In some cases, the chest compression device 700 includes at least one input device 722. In various examples, the input device(s) 722 is configured to receive an input signal from a user 724, who may be a rescuer treating the subject 706. Examples of the input device(s) 722 include, for instance, at a keypad, a cursor control, a touch-sensitive display, a voice input device (e.g., a microphone), a haptic feedback device (e.g., a gyroscope), or any combination thereof. In various implementations, the processor(s) 714 generate the control signal based on the input signal. For instance, the processor(s) 714 generate the control signal to adjust a frequency of the compressions based on the chest compression device 700 detecting a selection by the user 724 of a user interface element displayed on a touchscreen or detecting the user 724 pressing a button integrated with an external housing of the chest compression device 700.

According to some examples, the input device(s) 722 include one or more sensors. The sensor(s), for example, is configured to detect a physiological parameter of the subject 706. In some implementations, the sensor(s) is configured to detect a state parameter of the chest compression device 700, such as a position of the compressor 702 with respect to the subject 706 or the backplate 712, a force administered by the compressor 702 on the subject 706, a force administered onto the backplate 712 by the body of the subject 706 during a compression, or the like. According to some implementations, the signals transmitted by the transceiver(s) 716 indicate the physiological parameter(s) and/or the state parameter(s).

The chest compression device 700 further includes at least one output device 725, in various implementations. Examples of the output device(s) 725 include, for instance, least one of a display (e.g., a projector, an LED screen, etc.), a speaker, a haptic output device, a printer, or any combination thereof. In some implementations, the output device(s) 725 include a screen configured to display various parameters detected by and/or reported to the chest compression device 700, a charge level of the power source 708, a timer indicating a time since compressions were initiated or paused, and other relevant information.

The chest compression device 700 further includes memory 726. In various implementations, the memory 726 is volatile (such as random access memory (RAM)), nonvolatile (such as read only memory (ROM), flash memory, etc.) or some combination of the two. The memory 726 stores instructions that, when executed by the processor(s) 714, causes the processor(s) 714 to perform various operations. In various examples, the memory 726 stores methods, threads, processes, applications, objects, modules, any other sort of executable instruction, or a combination thereof. In some cases, the memory 726 stores files, databases, or a combination thereof. In some examples, the memory 726 includes, but is not limited to, RAM, ROM, EEPROM, flash memory, or any other memory technology. In some examples, the memory 726 includes one or more of CD-ROMs, DVDs, CAM, or other optical storage, magnetic cassettes, magnetic tape, magnetic disk storage or other magnetic storage devices, or any other medium which can be used to store the desired information. In various cases, the memory 726 stores instructions, programs, threads, objects, data, or any combination thereof, that cause the processor(s) 714 to perform various functions. In various cases, the memory 726 stores one or more parameters that are detected by the chest compression device 700 and/or reported to the chest compression device 700.

In some implementations, the memory 726 includes a modifier 728 that, when executed by the processor(s) 714, causes the processor(s) 714 to change a parameter of chest compressions applied to the subject 706. For instance, the modifier 728 includes instructions for identifying a sensitive condition of the subject 706 and/or generating an instruction to change a chest compression parameter. The instruction, for instance, is output to the user 724 via the output device(s) 725. In some cases, the processor(s) 714 causes the motor 704 and/or compressor 702 to change the chest compression parameter based on instructions specified by the modifier 728.

### EXAMPLE CLAUSES

The following clauses provide various implementations of the present disclosure. However, the scope of the disclosure is not limited to any of the clauses listed below.
1. A system, including: a defibrillator including: a detection circuit configured to detect an electrical signal indicative of an electrocardiogram (ECG) of a subject; a first processor configured to determine that the ECG is indicative of ventricular fibrillation (VF); and a treatment circuit configured to output an electrical shock to the subject in response to the first processor determining that the ECG is indicative of the VF; and a chest compression device communicatively coupled with the defibrillator, the chest compression device including: a compressor configured to administer compressions to a chest of the subject; and a second processor configured to: determine, by analyzing the ECG, that the subject has transitioned into the VF from a non-VF heart rhythm within a threshold time period after an initial compression among the compressions; and in response to determining that the subject has refibrillated by transitioning into the VF from the non-VF heart rhythm within a threshold time period after the initial compression, cause the compressor to decrease a rate of the compressions.
2. The system of clause 1, wherein the second processor is further configured to: in response to determining that the subject has refibrillated by transitioning into the VF from the non-VF heart rhythm within a threshold time period after the initial compression, cause the compressor to: reduce a depth of the compressions on the chest of the subject.
3. The system of clause 1 or 2, wherein the second processor is further configured to: in response to determining that the subject has refibrillated by transitioning into the VF from the non-VF heart rhythm within a threshold time period after the initial compression, cause the compressor to: increase a contact area of the compressions on the chest of the subject, in particular during a compression phase; and increase a height of the compressions on the chest of the subject during a decompression phase.
4. A medical device, including: a compressor configured to administer compressions to a chest of a subject; and a processor configured to: identify an electrocardiogram (ECG) of the subject; determine, by analyzing the ECG, that the subject has refibrillated; and in response to determining that the subject has refibrillated, cause the compressor to change a rate of the compressions.
5. The medical device of clause 4, wherein the compressor includes a plunger or a belt.
6. The medical device of clause 4 or 5, wherein the processor is configured to determine, by analyzing the ECG, that the subject has refibrillated by: determining that the ECG is indicative of a transition into ventricular fibrillation (VF) from a non-VF heart rhythm.
7. The medical device of clause 6, wherein the processor is further configured to determine that the transition occurred within a threshold time period after an initial compression among the compressions has been administered to the chest of the subject.
8. The medical device of any of clauses 4 to 7, wherein the processor is configured to cause the compressor to change the rate of the compressions by causing the compressor to decrease a duty cycle of the compressions.
9. The medical device of any of clauses 4 to 8, wherein the processor is configured to cause the compressor to change the rate of the compressions by causing the compressor to decrease the rate of the compressions.
10. The medical device of any of clauses 4 to 9, further including: a detection circuit configured to detect, from a heart of the subject, an electrical signal indicative of the ECG.
11. The medical device of any of clauses 4 to 10, further including: a transceiver configured to receive, from an external device, data indicative of the ECG.
12. The medical device of any of clauses 4 to 11, wherein the processor is further configured to: in response to determining that the subject has refibrillated, cause the compressor to increase a contact area of the compressor on the chest of the subject; and/or change a position of the compressions from a location on the chest of the subject to a location on an abdomen of the subject.
13. A method, including: administering first compressions to a chest of a subject at a first rate; identifying an ECG of the subject; determining, by analyzing the ECG, that the subject has refibrillated; and in response to determining that the subject has refibrillated, administering second compressions to the chest of the subject at a second rate, the second rate being different than the first rate.
14. The method of clause 13, wherein determining, by analyzing the ECG, that the subject has refibrillated includes: determining that the ECG is indicative of a transition into VF from a non-VF heart rhythm.
15. The method of clause 14, further including: determining that the transition occurred within a threshold time period after an initial compression among the first compressions has been administered to the chest of the subject.
16. The method of any of clauses 13 to 15, wherein the second rate is lower than the first rate.
17. The method of any of clauses 13 to 16, wherein a contact area of the first compressions on the chest is smaller than a contact area of the second compressions on the chest, and/or wherein a location of the first compressions on the body of the subject is different than a location of the second compressions on the body of the subject.
18. The method of any of clauses 13 to 17, wherein a depth of the first compressions is longer than a depth of the second compressions, and/or wherein a height of the first compressions is shorter than a height of the second compressions.
19. The method of any of clauses 13 to 18, wherein a duty cycle of the first compressions is higher than a duty cycle of the second compressions.
20. The method of any of clauses 13 to 19, further including: outputting, to the subject, a first electrical shock, wherein administering the first compressions to the chest of the subject at the first rate is in response to outputting the first electrical shock; and outputting, to the subject, a second electrical shock, wherein administering the second compressions to the chest of the subject at the second rate is in response to outputting the second electrical shock.
21. A system, including: a defibrillator including: a detection circuit configured to detect an electrical signal indicative of an electrocardiogram (ECG) of a subject, the ECG including a first segment, a second segment detected after the first segment, and a third segment detected after the second segment; a first processor configured to: determine that the first segment of the ECG is indicative of ventricular fibrillation (VF); determine that the second segment of the ECG is indicative of a non-VF heart rhythm; and determine that the third segment of the ECG is indicative of VF; and a treatment circuit configured to: output a first electrical shock to the subject in response to the first processor determining that the first segment of the ECG is indicative of VF; and output a second electrical shock to the subject in response to the first processor determining that the second segment of the ECG is indicative of VF; and a chest compression device communicatively coupled with the defibrillator, the chest compression device including: a compressor configured to administer compressions to a chest of the subject; and a second processor configured to: in response to the treatment circuit outputting the second electrical shock, cause the compressor to decrease a duty cycle of the compressions.
22. The system of clause 21, wherein the second processor is further configured to: in response to the treatment circuit outputting the second electrical shock, cause the compressor to: reduce a depth of the compressions on the chest of the subject; or increase a height of the compressions on the chest of the subject.
23. The system of clause 21 or 22, wherein the second processor is further configured to: in response to the treatment circuit outputting the second electrical shock, cause the compressor to: increase a contact area of the compressions on the chest of the subject; and/or change a location of the compressions on the body of the subject.
24. A medical device, including: a compressor configured to administer compressions to a chest of a subject receiving a first electrical shock and a second electrical shock; and a processor configured to: determine that the subject has received the second electrical shock within a threshold time period after the compressor administers the compressions; and in response to determining that the subject has received the second electrical shock within a threshold time period after the compressor administers the compressions, cause the compressor to change a duty cycle of the compressions.
25. The medical device of clause 24, wherein the compressor includes a plunger or a belt.
26. The medical device of clause 24 or 25, wherein the processor is configured to cause the compressor to change the duty cycle of the compressions by causing the compressor to decrease a duty cycle of the compressions.
27. The medical device of any of clauses 24 to 26, wherein the processor is configured to determine that the subject has received the second electrical shock within the threshold time period after the compressor administers the compressions by determining that the subject has received the electrical shock within the threshold time period after the compressor administers an initial compression among the compressions.
28. The medical device of any of clauses 24 to 27, further including: a sensor configured to detect the first electrical shock and the second electrical shock.
29. The medical device of any of clauses 24 to 28, further including: a detection circuit configured to detect, from a heart of the subject, an electrical signal indicative an ECG of the subject, wherein the processor is further configured to determine, by analyzing the ECG, that the subject has transitioned into VF from a non-VF heart rhythm within a threshold time period after the compressor administers the compressions.
30. The medical device of any of clauses 24 to 29, further including: a transceiver configured to receive, from an external device, a communication signal indicating the second electrical shock.
31. The medical device of any of clauses 24 to 30, wherein the processor is further configured to: in response to determining that the subject has received the second electrical shock within a threshold time period after the compressor administers the compressions, cause the compressor to increase a contact area of the compressor on the chest of the subject; and/or to change a location of the compressor on the body of the subject.
32. The medical device of any of clauses 24 to 31, wherein the processor is further configured to: in response to determining that the subject has received the second electrical shock within a threshold time period after the compressor administers the compressions, cause the compressor to decrease a frequency of the compressions.
33. A method, including: determining that a subject has received a first electrical shock; administering first compressions to a chest of a subject at a first duty cycle; in response to administering the first compressions to the chest of the subject at the first duty cycle, determining that the subject has received a second electrical shock; and in response to determining that the subject has received the second electrical shock, administering second compressions to the chest of the subject at a second duty cycle, the second duty cycle being different than the first duty cycle.
34. The method of clause 33, wherein determining that the subject has received a second electrical shock includes: receiving, from an external device, a communication signal indicating that an ECG of the subject is indicative of a transition into VF from a non-VF heart rhythm.
35. The method of clause 34, wherein the communication signal further indicates that the transition occurred within a threshold time period after an initial compression among the first compressions has been administered to the chest of the subject.
36. The method of any of clauses 33 to 35, wherein the second duty cycle is lower than the first duty cycle.
37. The method of any of clauses 33 to 36, wherein a contact area of the first compressions on the chest is smaller than a contact area of the second compressions on the chest.
38. The method of any of clauses 33 to 37, wherein a depth of the first compressions is longer than a depth of the second compressions and/or wherein a height of the first compressions is shorter than a height of the second compressions.
39. The method of any of clauses 33 to 38, wherein a frequency of the first compressions is higher than a frequency of the second compressions.
40. The method of any of clauses 33 to 39, wherein determining that the subject has received the first electrical shock includes detecting the first electrical shock or receiving a first communication signal indicating the first electrical shock, and wherein determining that the subject has received the second electrical shock includes detecting the second electrical shock or receiving a second communication signal indicating the second electrical shock.

### CONCLUSION

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be used for realizing implementations of the disclosure in diverse forms thereof.

As will be understood by one of ordinary skill in the art, each implementation disclosed herein can comprise, consist essentially of or consist of its particular stated element, step, or component. Thus, the terms "include" or "including" should be interpreted to recite: "comprise, consist of, or consist essentially of." The transition term "comprise" or "comprises" means has, but is not limited to, and allows for the inclusion of unspecified elements, steps, ingredients, or components, even in major amounts. The transitional phrase "consisting of" excludes any element, step, ingredient or component not specified. The transition phrase "consisting essentially of" limits the scope of the implementation to the specified elements, steps, ingredients or components and to those that do not materially affect the implementation. As used herein, the term "based on" is equivalent to "based at least partly on," unless otherwise specified.

Unless otherwise indicated, all numbers expressing quantities, properties, conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques. When further clarity is required, the term "about" has the meaning reasonably ascribed to it by a person skilled in the art when used in conjunction with a stated numerical value or range, i.e. denoting somewhat more or somewhat less than the stated value or range, to within a range of ±20% of the stated value; ±19% of the stated value; ±18% of the stated value; ±17% of the stated value; ±16% of the stated value; ±15% of the stated value; ±14% of the stated value; ±13% of the stated value; ±12% of the stated value; ±11% of the stated value; ±10% of the stated value; ±9% of the stated value; ±8% of the stated value; ±7% of the stated value; ±6% of the stated value; ±5% of the stated value; ±4% of the stated value; ±3% of the stated value; ±2% of the stated value; or ±1% of the stated value.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing implementations (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate implementations of the disclosure and does not pose a limitation on the scope of the disclosure. No language in the specification should be construed as indicating any non-claimed element essential to the practice of implementations of the disclosure.

Groupings of alternative elements or implementations disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain implementations are described herein, including the best mode known to the inventors for carrying out implementations of the disclosure. Of course, variations on these described implementations will become apparent to those of ordinary skill in the art upon reading the foregoing description. The inventors expect skilled artisans to employ such variations as appropriate, and the inventors intend for implementations to be practiced otherwise than specifically described herein. Accordingly, the scope of this disclosure includes all modifications and equivalents of the subject matter recited in the claims appended hereto as permitted by applicable law. Moreover, any combination of the above-described elements in all possible variations thereof is encompassed by implementations of the disclosure unless otherwise indicated herein or otherwise clearly contradicted by context.

## Claims

1. A medical device, comprising:
a compressor configured to administer compressions to a chest of a subject; and
a processor configured to:
identify an electrocardiogram (ECG) of the subject;
determine, by analyzing the ECG, that the subject has refibrillated; and
in response to determining that the subject has refibrillated, cause the compressor to change a rate of the compressions.

2. The medical device of claim 1, wherein the compressor comprises a plunger or a belt.

3. The medical device of claim 1 or 2, wherein the processor is configured to determine, by analyzing the ECG, that the subject has refibrillated by:
determining that the ECG is indicative of a transition into ventricular fibrillation (VF) from a non-VF heart rhythm.

4. The medical device of claim 3, wherein the processor is further configured to determine that the transition occurred within a threshold time period after an initial compression among the compressions has been administered to the chest of the subject.

5. The medical device of any of claims 1 - **4,** wherein the processor is configured to cause the compressor to change the rate of the compressions by causing the compressor to decrease a duty cycle of the compressions.

6. The medical device of any of claims 1 - 5, wherein the processor is configured to cause the compressor to change the rate of the compressions by causing the compressor to decrease the rate of the compressions.

7. The medical device of any of claims 1 - 6, further comprising:
a detection circuit configured to detect, from a heart of the subject, an electrical signal indicative of the ECG.

8. The medical device of any of claims 1 - 7, further comprising:
a transceiver configured to receive, from an external device, data indicative of the ECG.

9. The medical device of any of claims 1 - 8, wherein the processor is further configured to:
in response to determining that the subject has refibrillated, cause the compressor to increase a contact area of the compressor on the chest of the subject.

10. The medical device of any of claims 1 - 9, wherein the processor is configured to:
determine that the subject has previously refibrillated; and
in response to determining that the subject has previously refibrillated, cause the compressor to change the administration of the compressions from administration at a first parameter to administration at a second parameter different from the first parameter.

11. The medical device of any of claims 1 - 9, wherein the processor is configured to:
determine that the subject has received multiple electrical shocks; and
in response to determining that the subject has received multiple electrical shocks, cause the compressor to change the administration of the compressions from administration at a first parameter to administration at a second parameter different from the first parameter.

12. The medical device of claim 10 or 11, wherein the first parameter is a first rate, wherein the second parameter is a second rate.

13. The medical device of claim 12, wherein the second rate is lower than the first rate.

14. The medical device of claim 10 or 11, wherein the first parameter is a first compression depth, wherein the second parameter is a second compression depth

15. The medical device of claim 14, wherein the second compression depth is shorter than the first compression depth.
